# EUROPEAN PATENT SPECIFICATION

(11) **EP 2 931 685 B1**
(45) Date of publication and mention of the grant of the patent: **26.09.2018**
(21) Application number: 13861871.5
(22) Date of filing: 15.11.2013
(51) Int. Cl.: C07C 5/13, C07C 5/367, C07C 13/10, C07C 15/02

(54) **METHODS AND APPARATUSES FOR INCREASING ALKYL-CYCLOPENTANE CONCENTRATIONS IN AROMATIC-RICH STREAMS**
VERFAHREN UND VORRICHTUNGEN FÜR ERHÖHTE ALKYL-CYCLOPENTAN-KONZENTRATIONEN IN AROMASTOFFREICHEN STRÖMEN
PROCÉDÉS ET APPAREILS POUR AUGMENTER LES CONCENTRATIONS EN ALKYL-CYCLOPENTANE DANS DES COURANTS RICHES EN PRODUITS AROMATIQUES

(30) Priority: 14.12.2012 US 201213715838
(43) Date of publication of application: 21.10.2015
(73) Proprietor: UOP LLC, Des Plaines, Illinois 60017-5017 (US)
(72) Inventor: GLOVER, Bryan K., Des Plaines, Illinois 60017-5017 (US)
(74) Representative: Boult Wade Tennant LLP
(86) International application number: PCT/US2013/070241
(87) International publication number: WO 2014/092928

(56) References cited:
- EP-A1- 1 598 411
- EP-A1- 1 992 673
- WO-A1-2011/096995
- US-A- 3 287 253
- US-A- 5 350 504
- US-A1- 2006 183 952
- US-A1- 2006 183 952
- SATOH DAISHI ET AL.: 'Isomerization of cycloheptane, cyclooctane, and cyclodecane catalyzed by sulfated zirconia - comparison with open-chain alkanes.' PHYS. CHEM. CHEM. PHYS. vol. 5, 2003, pages 4343 - 4349, XP055255743

## Description

### STATEMENT OF PRIORITY

This application claims priority to U.S. Application No. 13/715,838 which was filed on December 14, 2012.

### TECHNICAL FIELD

The technical field generally relates to methods for forming low-aromatic high-octane product streams from aromatic-rich streams, and more particularly relates to methods for increasing alkyl-cyclopentane concentrations in toluene-rich streams.

### BACKGROUND

Aromatics such as benzene and toluene are naturally-occurring constituents of crude oil and of many petroleum products. Aromatics have been added to gasoline blending to increase the gasoline's octane number, e.g., Research Octane Number (RON). However, due to harmful biological and environmental effects of aromatics, standards have been enacted to regulate their content in fuels such as gasoline. For example, certain standards may limit gasoline to concentrations of no more than 35 wt% aromatics. At the same time, fuel standards continue to require high octane numbers for gasoline.

The demand for distillates as fuel has been increasing and is forecasted to continue to grow strongly. Specifically, consumption of diesel and middle distillate components is projected to be the primary driver for oil consumption growth over the next decade. Industrial production of distillates is primarily supported by hydrocracking processes, rather than by fluid catalytic cracking (FCC). The growing demand for diesel and other distillates has led to the construction of refineries that rely on hydrocracking units designed to maximize production of distillates rather than fluid catalytic cracking units. Furthermore, new refineries often lack fluid catalytic cracking units altogether.

Blending of aromatic-rich streams into other hydrocarbon streams during processing of gasoline fuel blends is easily accomplished in refineries with fluid catalytic cracking units, as the fluid catalytic cracking process provides a source of moderately high octane and moderate aromatic blend material to compensate for the addition of aromatics. However, in refineries that rely on hydrocracking units and that lack fluid catalytic cracking units, the ratio of heavy naphtha reformate streams, high in aromatics, to light naphtha isomerate streams, void of aromatics, produced by hydrocracking is too high to provide economical gasoline blending with the required low aromatic levels and high octane numbers. For example, the gasoline range naphthas that come from crude units and hydrocracking units typically have a heavy naphtha reformate (C7+) to light naphtha isomerate (C5s and C6s) ratio of 2:1. The heavy naphtha reformate typically has a toluene concentration of 70-75 wt%. While it has been possible to blend the heavy naphtha and light naphtha to reach the minimum octane requirement, such blends typically cannot meet the required low aromatic concentration.

Therefore, there is a growing need in refineries that lack fluid catalytic cracking units, as well as in other processing schemes, for processing capabilities that reduce toluene levels in gasoline blends. Further, there is a growing need in those refineries for processing capabilities that increase octane numbers in gasoline blends. A method that both reduces toluene levels and avoids significant octane loss would provide a source for additional gasoline production.

Accordingly, it is desirable to provide novel methods and apparatuses for increasing alkyl-cyclopentane concentrations in aromatic-rich streams. It is also desirable to provide methods and apparatuses for processing hydrocarbon streams into gasoline blends. Also, it is desirable to provide such methods and apparatuses that operate economically. Furthermore, other desirable features and characteristics will become apparent from the subsequent detailed description and the appended claims, taken in conjunction with the accompanying drawings and the foregoing technical field and background.

US 2006/183952 A1 concerns a process for reducing the amount of benzene in a hydrocarbon stream, while improving the octane number. This process comprises the hydrogenation of the aromatic-containing stream in order to form cyclohexane, which is consequently isomerized to methyl cyclopentane.

### BRIEF SUMMARY

Methods and apparatuses for increasing an alkyl-cyclopentane concentration in an aromatic-rich stream and methods for processing hydrocarbons are provided. In one exemplary embodiment, a method for increasing an alkyl-cyclopentane concentration in an aromatic-rich stream includes saturating aromatics in the aromatic-rich stream to form methylcyclohexane. Further, the method includes isomerizing the methylcyclohexane to form alkyl-cyclopentanes. The method dehydrogenates residual methylcyclohexane to form aromatics in a product stream. The product stream includes aromatics and alkyl-cyclopentanes.

In another embodiment, a method for processing hydrocarbons is provided. The method for processing hydrocarbons includes providing a toluene-rich stream. Further, the method includes converting toluene in the toluene-rich stream into methylcyclohexane. In the method, a portion of the methylcyclohexane is converted into alkyl-cyclopentanes.

An apparatus for increasing an alkyl-cyclopentane concentration in a toluene-rich stream is described herein. The apparatus includes a saturation zone configured to receive the toluene-rich stream and to saturate toluene therein to form a saturated stream comprising toluene and methylcyclohexane. The apparatus also includes an isomerization zone configured to receive the saturated stream and to isomerize the methylcyclohexane therein to form an isomerized stream comprising toluene, methylcyclohexane and alkyl-cyclopentanes. The apparatus further includes a dehydrogenation zone configured to receive the isomerized stream and to dehydrogenate the methylcyclohexane therein to form a product stream comprising toluene and alkyl-cyclopentanes.

### BRIEF DESCRIPTION OF THE DRAWINGS

Methods and apparatuses for increasing alkyl-cyclopentane concentrations in hydrocarbon streams will hereinafter be described in conjunction with the following drawing figures, wherein like numerals denote like elements, and wherein:
FIG. 1 is a schematic diagram of an embodiment of an apparatus and method for increasing alkyl-cyclopentane concentrations in hydrocarbon streams including a saturation zone, an isomerization zone, and a dehydrogenation zone in accordance with an embodiment; and
FIG. 2 is a schematic diagram of an embodiment of an apparatus and method for increasing alkyl-cyclopentane concentrations in hydrocarbon streams including a combined saturation/isomerization zone and a dehydrogenation zone in accordance with an embodiment.

### DETAILED DESCRIPTION

The following detailed description is merely exemplary in nature and is not intended to limit the methods for increasing alkyl-cyclopentane concentrations in aromatic-rich streams. Furthermore, there is no intention to be bound by any theory presented in the preceding background or the following detailed description.

Methods for processing hydrocarbons, and more particularly, for increasing alkyl-cyclopentane concentrations in aromatic-rich streams are provided herein. The methods enable hydrocarbon product streams to be obtained with low levels of aromatics, such as no more than 35 wt% aromatics, while achieving high octane numbers, such as a RON of 95. The low aromatic concentration and high octane number is achieved by converting a portion of the aromatics to high octane number alkyl-cyclopentanes to increase the alkyl-cyclopentane concentration of the gasoline blend while simultaneously decreasing the aromatic concentration. In an exemplary embodiment, the portion of aromatics is converted to dimethylcyclopentane, as dimethylcyclopentane has an unusually high octane number in comparison to other C7 species. Certain embodiments convert a portion of a toluene content to high octane number alkyl-cyclopentanes to increase the alkyl-cyclopentane concentration of the gasoline blend while simultaneously decreasing the toluene concentration.

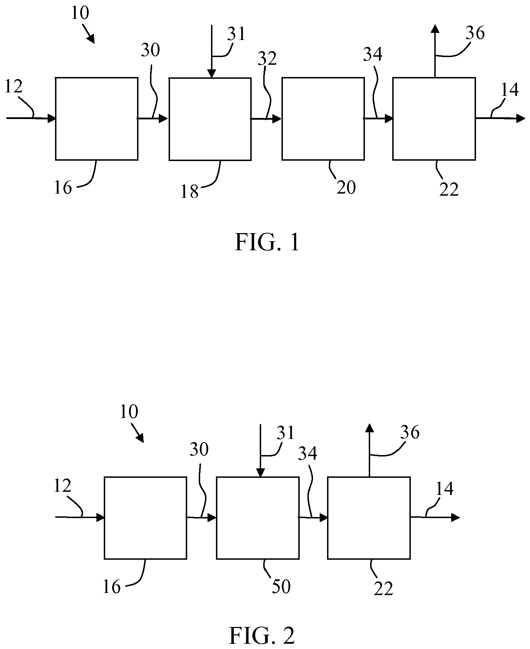

In an embodiment, and as shown in FIG. 1, an apparatus 10 for increasing alkyl-cyclopentane concentrations in hydrocarbon streams receives and processes a hydrocarbon stream 12 or feedstock to form a product stream 14 including aromatics, for example toluene, and alkyl-cyclopentanes.

The apparatus 10 includes a processing zone 16, a saturation zone 18, an isomerization zone 20, and a dehydrogenation zone 22. As shown, the processing zone 16 receives the hydrocarbon stream 12. Suitable hydrocarbon streams 12 include natural gasoline, straight run naphtha, and refined feedstocks. The processing zone 16 may comprise a fractionation unit, a hydrocracking unit, a reforming unit, or other processing unit that forms an aromatic-rich stream 30, such as a toluene-rich stream. For example, the processing unit 16 may be a fractionation unit for fractionating the aromatic-rich stream 30 from a catalytic reformate. Or, the processing zone 16 could include a toluene extraction unit forming a toluene-rich stream 30, though such a unit may be uneconomical for the purposes described herein. The phrases "aromatic-rich" and "toluene-rich" as used herein are not intended to require any minimum concentration of aromatics or toluene in the referenced stream other than above zero. As such, "aromatic-rich" and "toluene-rich" may be interpreted as "aromatic-containing" and "toluene-containing" herein. However, economic use of the apparatus and method is envisioned as being supported at higher aromatic, or specifically, toluene concentrations. In certain embodiments, the aromatic-rich stream may comprise over 70 wt% aromatics [based on the total weight of the stream 30], such as 80-85 wt% aromatics. Likewise, a toluene-rich stream may comprise over 70 wt% toluene [based on the total weight of the stream 30], such as 80-85 wt% toluene.

As shown in FIG. 1, the aromatic-rich stream 30 is received by saturation zone 18. In saturation zone 18, the double bonds (or aromatic bonds) in toluene, or other aromatics, are saturated or hydrogenated with hydrogen during processing. For that purpose, the exemplary saturation zone 18 includes a saturation reactor that holds a fixed bed of catalyst for promoting the saturation/hydrogenation of toluene or other aromatics. In an exemplary embodiment, the saturation zone 18 includes a non-acidic catalyst that supports an almost exclusive conversion of toluene or other aromatics into methylcyclohexane, rather than to alkyl-cyclopentanes. Known saturation/hydrogenation catalysts may be used for this process. For example, an H-8 catalyst may be used. Suitable saturation/hydrogenation catalysts will provide a metallic function to promote hydrogen transfer without any substantial acid function that would lead to undesirable cracking. Saturation/hydrogenation catalyst compositions may include platinum group, tin, nickel, or cobalt and molybdenum metals on suitable refractory inorganic oxide supports such as alumina. The alumina may be an anhydrous gamma-alumina with a high degree of purity. The term "platinum group metals" refers to noble metals excluding silver and gold that are selected from the group consisting of platinum, palladium, germanium, ruthenium, rhodium, osmium, and iridium. Such saturation/hydrogenation catalysts will provide satisfactory aromatic saturation at the operating conditions including temperatures of from 250°C to 320°C. An exemplary saturation/hydrogenation catalyst is a noble metal catalyst that is selective and has no measureable side reactions. With the appropriate saturation/hydrogenation catalyst, no cracking of the hydrocarbons occurs and no coke forms on the saturation/hydrogenation catalyst to reduce activity.

Saturation zone 18 is configured to saturate or hydrogenate toluene or other aromatics in the aromatic-rich stream 30 with hydrogen from a hydrogen stream 31 to form cyclohexanes. More specifically, saturation zone 18 is configured to partially saturate the aromatic-rich stream 30 by saturating or hydrogenating a portion of the toluene or other aromatics therein to cyclohexanes. During an exemplary saturation/hydrogenation process toluene is converted to methylcyclohexane as illustrated:

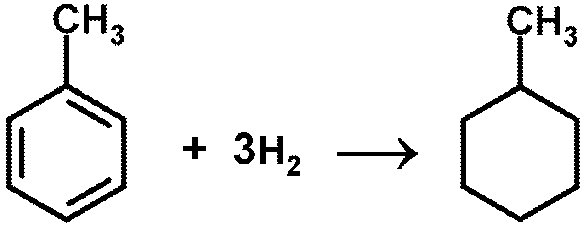

As shown in FIG. 1, hydrogen 31 is delivered directly to the saturation zone 18; however, it is also contemplated that the hydrogen 31 may be combined with the aromatic-rich stream 30 upstream of the saturation zone 18. In an exemplary embodiment, a slight excess of hydrogen 31 above the stoichiometric level is provided. For toluene saturation/hydrogenation, three moles of hydrogen are required for each mole of toluene saturated. Within the saturation zone 18, the double bonds in toluene in the aromatic-rich stream 30 are saturated with hydrogen 31 at moderate process conditions. As a result of saturation of aromatic bonds, toluene is converted to methylcyclohexane. As the toluene-methylcyclohexane equilibrium is strongly influenced by temperature and pressure, saturation/hydrogenation reaction conditions must be selected and monitored carefully. The saturation/hydrogenation process is highly exothermic and the high heat of reaction associated with toluene saturation/hydrogenation is managed to control the temperature rise across the saturation zone 18.

Because the saturation reaction is highly exothermic, the heat release may require special treatment. For example, the saturation zone 18 may include sequential stages with inter stage cooling. Alternatively, the saturation zone 18 may utilize a recycle/diluent to manage heat. Or the saturation zone 18 may use a temperature controlled saturation reactor, such as a liquid cooled reactor, a steam generating reactor, or other known systems.

In a saturation zone using more than one saturation reactor in series, particularly those using adiabatic saturation reactors connected in series, it will normally be necessary to control the temperature rise across each catalyst bed in order to avoid hot spot formation and to obviate the risk of temperature runaways occurring. It is typically disadvantageous to operate with too high an exit temperature from the catalyst bed because high exit temperatures often result in increased formation of by-products. One way of controlling the temperature rise across a catalyst bed of an adiabatic reactor is recycle product liquid to absorb heat. Another way is to increase the amount of hydrogen in the circulating gas or to allow inert gases, such as nitrogen, to build up in the recirculating gas. In this way the extra gas acts as a heat sink to absorb the exothermic heat of reaction. However, the size of the gas conduits and the size of the gas recycle compressor must be increased to allow for the increased gas flow and the capital costs of the plant are increased. Use of a plurality of adiabatic reactors in series with injection of cold shots of gas between reactors is another solution. Inter-bed cooling is another method of limiting the temperature rise across an adiabatically operated catalyst bed.

By partially saturating the aromatic-rich stream 30, the saturation zone 18 forms a saturated stream 32 comprised of toluene or other aromatics and methylcyclohexane. In an exemplary embodiment, high toluene or other aromatics saturation is achieved, such as 90% or more. In other embodiments, a lower saturation may be achieved, particularly in cases where maximum octane is to be maintained.

In FIG. 1, the saturated stream 32 is received by the isomerization zone 20. The isomerization zone 20 is configured to isomerize methylcyclohexane in the saturated stream 32 to form alkyl-cyclopentanes. Specifically, a portion of the methylcyclohexane is isomerized over a catalyst to alkyl-cyclopentanes. For example, between 30 wt% and 40 wt% of the methylcyclohexane may be converted into alkyl-cyclopentanes. In an exemplary embodiment, the methylcyclohexane is isomerized to dimethylcyclopentane, according to the reaction:

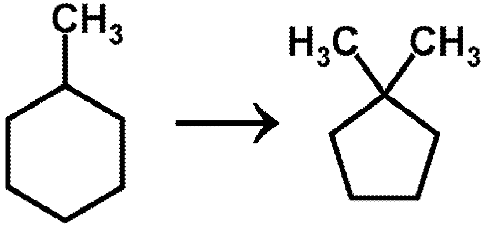

While 1,1 dimethylcyclopentane is illustrated, other dimethylcyclopentanes may be formed. Formation of alkyl-cyclopentanes is favored at high temperatures, such as above 200°C. Operating conditions within the isomerization zone 20 are selected to maximize the production of alkyl-cyclopentanes from methylcyclohexanes introduced therein, and may be selected to maximize the production of dimethylcyclopentane or of a particular dimethylcyclopentane. Operating conditions within the isomerization zone 20 are dependent upon various factors including, but not limited to, feed severity and catalyst type, and those of skill in the art are readily able to identify appropriate operating conditions within the isomerization zone 20 to maximize formation of alkyl-cyclopentanes in general and dimethylcyclopentanes specifically from the isomerization of methylcyclohexane.

The isomerization catalyst can be a conventional paraffin isomerization catalyst or a lower acidity metal-containing catalyst. For example, the isomerization catalyst can comprise a Group VIII metal, such as platinum or palladium, on a porous inorganic oxide support, for example alumina, silica/alumina or an alumino-silicate such as a zeolite. Suitable isomerization catalysts comprise sulfated zirconia, platinum on chloride alumina and platinum on a zeolite. Suitable zeolites include faujasite, mordenite and synthetic alumino-silicates.

As a result of isomerization, the isomerization zone 20 forms an isomerized stream 34 comprising alkyl-cyclopentanes, toluene or other aromatics and methylcyclohexane. In an exemplary embodiment, the isomerized stream 34 includes no more than 35 wt% residual methylcyclohexane.

As shown in FIG. 1, the isomerized stream 34 is received by the dehydrogenation zone 22. The dehydrogenation zone 22 is configured to dehydrogenate the residual methylcyclohexane in the isomerized stream 34 to form toluene according to the reaction:

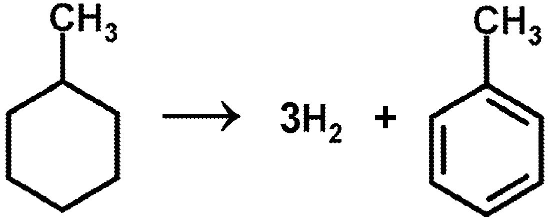

This reaction is performed over a catalyst, such as a mono-functional dehydrogenation catalyst. Mono and bi-metallic platinum catalysts may be used. The dehydrogenation reaction is endothermic and may be heated through heat exchange with streams from the saturation zone 18. During dehydrogenation, substantially all of the methylcyclohexane is converted to toluene. Further, a hydrogen stream 36 is formed and exits the dehydrogenation zone 22. The hydrogen stream 36 may be recycled for use as the hydrogen stream 31 entering the saturation zone 18.

As a result of the dehydrogenation step, the product stream 14 is formed by the dehydrogenation zone 22 and contains alkyl-cyclopentanes, such as dimethylcyclopentane, and toluene or other aromatics. The product stream 14 may also contain residual methylcyclohexane. As described herein, the product stream 14 includes an increased concentration of alkyl-cyclopentanes as compared to the aromatic-rich stream 30. For example, the product stream 14 may include over 30 wt% alkyl-cyclopentanes, such as over 50 wt% alkyl-cyclopentanes, or for example over 60 wt% alkyl-cyclopentanes. In an exemplary embodiment, the product stream 14 includes 65 wt% alkyl-cyclopentanes and 35 wt% toluene or other aromatics. Such a stream has an octane number (RON) of 95 and meets the gasoline standard of no more than 35 wt% aromatics. Of course, the exact composition of the product stream 14 is dependent on the composition of the aromatic-rich stream 30 and on processing conditions in the saturation zone 18, isomerization zone 20 and dehydrogenation zone 22, which can be manipulated to reach a desired toluene or other aromatics and alkyl-cyclopentane concentration. Nevertheless, the apparatus 10 and method described herein can provide for the economical production of the product stream 14 appropriate for use as gasoline with a required low aromatic content, such as lower than 35 wt%, and high octane number, such as higher than 90 or 95. In an embodiment with an initial amount X of toluene or other aromatics in the aromatic-rich stream, the apparatus and method can form the product stream with 0.65X dimethylcyclopentane and 0.35X toluene or other aromatics.

Referring to FIG. 2, an alternative apparatus 10 for increasing the alkyl-cyclopentane concentration in an aromatic-rich stream is illustrated. In FIG. 2, the processing zone 16 again forms an aromatic-rich stream 30 from a hydrocarbon stream. The details of the processing zone 16 are not described herein, but the processing zone 16 can include any hydrocarbon processing unit that forms an aromatic-rich stream, such as a hydrocracking unit, a reforming unit, or an aromatic or toluene extraction unit.

As shown, a combined saturation/isomerization zone 50 receives the aromatic-rich stream 30 and a hydrogen stream 31. The combined saturation/isomerization zone 50 includes a bifunctional catalyst that catalyzes both the saturation/hydrogenation of toluene or other aromatics to form methylcyclohexane and the isomerization of methylcyclohexane to alkyl-cyclopentanes, such as dimethylcyclopentane. As shown, the combined saturation/isomerization zone 50 forms an isomerized stream 34. In an exemplary embodiment, the combined saturation/isomerization zone 50 forms an isomerized stream 34 comprising 65 wt% alkyl-cyclopentanes, such as dimethylcyclopentane, and 35 wt% methylcyclohexane and toluene or other aromatics.

As shown in FIG. 2, the isomerized stream 34 is received by the dehydrogenation zone 22. The dehydrogenation zone 22 is configured to dehydrogenate the residual methylcyclohexane in the isomerized stream 34 over a catalyst to form toluene. An exemplary catalyst is a mono-functional dehydrogenation catalyst. Mono and bi-metallic platinum catalysts may be used. The dehydrogenation reaction is endothermic and may be heated through heat exchange with streams from the saturation zone 18. During dehydrogenation, substantially all of the methylcyclohexane is converted to toluene. Further, a hydrogen stream 36 is formed and exits the dehydrogenation zone 22. The hydrogen stream 36 may be recycled for use as the hydrogen stream 31 entering the combined saturation/isomerization zone 50.

As a result of the dehydrogenation step, the product stream 14 is formed by the dehydrogenation zone 22 and contains alkyl-cyclopentanes, such as dimethylcyclopentane, and toluene. The product stream 14 may also contain residual methylcyclohexane. As described herein, the product stream 14 includes an increased concentration of alkyl-cyclopentanes as compared to the aromatic-rich stream 30. For example, the product stream 14 may include over 30 wt% alkyl-cyclopentanes, such as over 50 wt% alkyl-cyclopentanes, or for example over 60 wt% alkyl-cyclopentanes. In an exemplary embodiment, the product stream 14 includes 65 wt% alkyl-cyclopentanes and 35 wt% toluene. Such a stream has an octane number of 95 and meets the gasoline standard of no more than 35 wt% aromatics. Of course, the exact composition of the product stream 14 is dependent on the composition of the aromatic-rich stream 30 and on processing conditions in the combined saturation/isomerization zone 50 and dehydrogenation zone 22 which can be manipulated to reach a desired toluene or other aromatics and alkyl-cyclopentane concentration. As described, the apparatus 10 and method can provide for the economical production of the product stream 14 for use as gasoline with a required low aromatic content and high octane number.

As described herein, an apparatus and method for increasing an alkyl-cyclopentane concentration in an aromatic-rich stream have been provided. In exemplary embodiments, an apparatus and method have been described for increasing a dimethylcyclopentane concentration in an aromatic-rich stream. The apparatus and method described above are particularly well-suited for the formation of a gasoline product stream that has both a low aromatic level, such as less than 35 wt%, and a high octane number, such as at least 95.

While at least one exemplary embodiment has been presented in the foregoing detailed description, it should be appreciated that a vast number of variations exist. It should also be appreciated that the exemplary embodiment or exemplary embodiments are only examples, and are not intended to limit the scope, applicability, or configuration of the claimed subject matter in any way. Rather, the foregoing detailed description will provide those skilled in the art with a convenient road map for implementing an exemplary embodiment or embodiments. It being understood that various changes may be made in the function and arrangement of elements described in an exemplary embodiment without departing from the scope set forth in the appended claims.

### SPECIFIC EMBODIMENTS

While the following is described in conjunction with specific embodiments, it will be understood that this description is intended to illustrate and not limit the scope of the appended claims.

A first embodiment of the invention is a method for increasing an alkyl-cyclopentane concentration in an aromatic-rich stream comprising
saturating aromatics in the aromatic-rich stream to form alkyl-cyclohexane;
wherein saturating aromatics in the aromatic-rich stream to form alkyl-cyclohexane comprises saturating aromatics in the aromatic-rich stream over a non-acidic catalyst to form alkyl-cyclohexane;
isomerizing the alkyl-cyclohexane to form alkyl-cyclopentanes; and
dehydrogenating residual alkyl-cyclohexane to form aromatics in a product stream, wherein the product stream includes aromatics and the alkyl-cyclopentanes;
wherein the aromatic-rich stream has an initial concentration of 60 wt% aromatics, and wherein dehydrogenating residual alkyl-cyclohexane to form aromatics in a product stream forms the product stream with less than 25 wt% aromatics.
An embodiment of the invention is one, any or all of prior embodiments in this paragraph up through the first embodiment in this paragraph wherein isomerizing the alkyl-cyclohexane to form alkyl-cyclopentanes comprises isomerizing the alkyl-cyclohexane to form dimethylcyclopentane. An embodiment of the invention is one, any or all of prior embodiments in this paragraph up through the first embodiment in this paragraph wherein saturating aromatics in the aromatic-rich stream to form alkyl-cyclohexane and isomerizing the alkyl-cyclohexane to form alkyl-cyclopentanes comprise simultaneously saturating aromatics in the aromatic-rich stream and isomerizing alkyl-cyclohexane over a bifunctional catalyst to form alkyl-cyclopentane. An embodiment of the invention is one, any or all of prior embodiments in this paragraph up through the first embodiment in this paragraph wherein dehydrogenating residual alkyl-cyclohexane to form aromatics in a product stream comprises dehydrogenating the residual alkyl-cyclohexane over a mono-functional dehydrogenation catalyst. An embodiment of the invention is one, any or all of prior embodiments in this paragraph up through the first embodiment in this paragraph further comprising fractionating the aromatic-rich stream using a catalytic reformate. An embodiment of the invention is one, any or all of prior embodiments in this paragraph up through the first embodiment in this paragraph further comprising fractionating the aromatic-rich stream using a catalytic reformate, wherein the aromatic-rich stream comprises at least 15 wt% toluene, wherein toluene is saturated in the aromatic-rich stream to form methylcyclohexane, and wherein residual methylcyclohexane is dehydrogenated to form toluene in the product stream. An embodiment of the invention is one, any or all of prior embodiments in this paragraph up through the first embodiment in this paragraph wherein dehydrogenating residual methylcyclohexane to form toluene in a product stream forms the product stream with an octane number (RON) of at least 90. An embodiment of the invention is one, any or all of prior embodiments in this paragraph up through the first embodiment in this paragraph wherein dehydrogenating residual methylcyclohexane to form toluene in a product stream forms the product stream with an octane number (RON) of at least 95.

A second embodiment of the invention is a method for processing hydrocarbons comprising providing a toluene-rich stream; converting toluene in the toluene-rich stream into methylcyclohexane; and converting a portion of the methylcyclohexane into alkyl-cyclopentanes. An embodiment of the invention is one, any or all of prior embodiments in this paragraph up through the second embodiment in this paragraph wherein converting a portion of the methylcyclohexane into alkyl-cyclopentanes comprises converting more than 30 wt% of the methylcyclohexane into alkyl-cyclopentanes. An embodiment of the invention is one, any or all of prior embodiments in this paragraph up through the second embodiment in this paragraph wherein converting a portion of the methylcyclohexane into alkyl-cyclopentanes comprises selectively converting more than 30 wt% of the methylcyclohexane into dimethylcyclopentane. An embodiment of the invention is one, any or all of prior embodiments in this paragraph up through the second embodiment in this paragraph wherein converting toluene in the toluene-rich stream into methylcyclohexane and converting a portion of the methylcyclohexane into alkyl-cyclopentanes comprise simultaneously saturating toluene in the toluene-rich stream and isomerizing methylcyclohexane over a bifunctional catalyst to form dimethylcyclopentane. An embodiment of the invention is one, any or all of prior embodiments in this paragraph up through the second embodiment in this paragraph wherein converting toluene in the toluene-rich stream into methylcyclohexane comprises saturating toluene in the toluene-rich stream over a non-acidic catalyst. An embodiment of the invention is one, any or all of prior embodiments in this paragraph up through the second embodiment in this paragraph wherein converting a portion of the methylcyclohexane into alkyl-cyclopentanes comprises isomerizing a portion of the methylcyclohexane to form dimethylcyclopentane. An embodiment of the invention is one, any or all of prior embodiments in this paragraph up through the second embodiment in this paragraph further comprising converting a remaining portion of the methylcyclohexane into toluene wherein converting a remaining portion of the methylcyclohexane into toluene comprises dehydrogenating the remaining portion of the methylcyclohexane over a mono-functional dehydrogenation catalyst. An embodiment of the invention is one, any or all of prior embodiments in this paragraph up through the second embodiment in this paragraph further comprising converting a remaining portion of the methylcyclohexane into toluene, wherein the toluene-rich stream comprises 60 wt% toluene and has an initial amount X of toluene, and wherein converting a remaining portion of the methylcyclohexane into toluene forms a stream with 0.65X alkyl-cyclopentanes and 0.35X toluene. An embodiment of the invention is one, any or all of prior embodiments in this paragraph up through the second embodiment in this paragraph further comprising converting a remaining portion of the methylcyclohexane into toluene, wherein converting a remaining portion of the methylcyclohexane into toluene forms a stream of toluene and alkyl-cyclopentanes having an octane number of at least 95.

## Claims

1. A method for increasing an alkyl-cyclopentane concentration in an aromatic-rich stream (30) comprising:
saturating aromatics in the aromatic-rich stream to form alkyl-cyclohexane;
wherein saturating aromatics in the aromatic-rich stream to form alkyl-cyclohexane comprises saturating aromatics in the aromatic-rich stream over a non-acidic catalyst to form alkyl-cyclohexane;
isomerizing the alkyl-cyclohexane to form alkyl-cyclopentanes; and
dehydrogenating residual alkyl-cyclohexane to form aromatics in a product stream (14), wherein the product stream includes aromatics and the alkyl-cyclopentanes;
wherein the aromatic-rich stream has an initial concentration of 60 wt% aromatics, and wherein dehydrogenating residual alkyl-cyclohexane to form aromatics in a product stream forms the product stream with less than 25 wt% aromatics.

2. The method of claim 1 wherein isomerizing the alkyl-cyclohexane to form alkyl-cyclopentanes comprises isomerizing the alkyl-cyclohexane to form dimethylcyclopentane.

3. The method of claim 1 wherein saturating aromatics in the aromatic-rich stream to form alkyl-cyclohexane and isomerizing the alkyl-cyclohexane to form alkyl-cyclopentanes comprise simultaneously saturating aromatics in the aromatic-rich stream and isomerizing alkyl-cyclohexane over a bifunctional catalyst to form alkyl-cyclopentane.

4. The method of claim 1 wherein dehydrogenating residual alkyl-cyclohexane to form aromatics in a product stream comprises dehydrogenating the residual alkyl-cyclohexane over a mono-functional dehydrogenation catalyst.

5. The method of claim 1 further comprising fractionating the aromatic-rich stream using a catalytic reformate.

6. The method of claim 1 further comprising fractionating the aromatic-rich stream using a catalytic reformate, wherein the aromatic-rich stream comprises at least 15 wt% toluene, wherein toluene is saturated in the aromatic-rich stream to form methylcyclohexane, and wherein residual methylcyclohexane is dehydrogenated to form toluene in the product stream.

7. The method of claim 6 wherein dehydrogenating residual methylcyclohexane to form toluene in a product stream forms the product stream with an octane number (RON) of at least 90.

## Patentansprüche

1. Verfahren zum Erhöhen einer Alkylcyclopentankonzentration in einem aromatenreichen Strom (30), umfassend:
Sättigen von Aromaten in dem aromatenreichen Strom, um Alkylcyclohexan zu erzeugen;
wobei das Sättigen von Aromaten in dem aromatenreichen Strom zum Erzeugen von Alkylcyclohexan das Sättigen von Aromaten in dem aromatenreichen Strom über einem nichtsauren Katalysator zum Erzeugen von Alkylcyclohexan umfasst;
Isomerisieren des Alkylcyclohexans, um Alkylcyclopentane zu bilden; und
Dehydrieren von restlichem Alkylcyclohexan, um Aromaten in einem Produktstrom (14) zu erzeugen, wobei der Produktstrom Aromaten und die Alkylcyclopentane enthält;
wobei der aromatenreiche Strom eine Anfangskonzentration von 60 Gew.-% Aromaten aufweist und wobei das Dehydrieren von restlichem Alkylcyclohexan zum Erzeugen von Aromaten in einem Produktstrom den Produktstrom mit weniger als 25 Gew.-% Aromaten bildet.

2. Verfahren gemäß Anspruch 1, wobei das Isomerisieren des Alkylcyclohexans zum Erzeugen von Alkylcyclopentanen das Isomerisieren des Alkylcyclohexans zum Erzeugen von Dimethylcyclopentan umfasst.

3. Verfahren gemäß Anspruch 1, wobei das Sättigen von Aromaten in dem aromatenreichen Strom zum Erzeugen von Alkylcyclohexan und das Isomerisieren des Alkylcyclohexans zum Erzeugen von Alkylcyclopentanen das gleichzeitige Sättigen von Aromaten in dem aromatenreichen Strom und Isomerisieren von Alkylcyclohexan über einem bifunktionellen Katalysator umfasst, um Alkylcyclopentan zu erzeugen.

4. Verfahren gemäß Anspruch 1, wobei das Dehydrieren von restlichem Alkylcyclohexan zum Erzeugen von Aromaten in einem Produktstrom das Dehydrieren des restlichen Alkylcyclohexans über einem monofunktionellen Dehydrierungskatalysator umfasst.

5. Verfahren gemäß Anspruch 1, ferner umfassend Fraktionieren des aromatenreichen Stroms unter Verwendung eines katalytischen Reformats.

6. Verfahren gemäß Anspruch 1, ferner umfassend Fraktionieren des aromatenreichen Stroms unter Verwendung eines katalytischen Reformats, wobei der aromatenreiche Strom wenigstens 15 Gew.-% Toluol umfasst, wobei das Toluol in dem aromatenreichen Strom gesättigt wird, um Methylcyclohexan zu erzeugen, und wobei restliches Methylcyclohexan dehydriert wird, um Toluol in dem Produktstrom zu erzeugen.

7. Verfahren gemäß Anspruch 6, wobei das Dehydrieren von restlichem Methylcyclohexan zum Erzeugen von Toluol in einem Produktstrom den Produktstrom mit einer Oktanzahl (RON) von wenigstens 90 bildet.

## Revendications

1. Méthode d'augmentation d'une concentration en alkyl-cyclopentane dans un courant riche en hydrocarbures aromatiques (30), comprenant :
la saturation d'hydrocarbures aromatiques dans le courant riche en hydrocarbures aromatiques afin de former de l'alkyl-cyclohexane ;
où la saturation d'hydrocarbures aromatiques dans le courant riche en hydrocarbures aromatiques afin de former de l'alkyl-cyclohexane comprend la saturation d'hydrocarbures aromatiques dans le courant riche en hydrocarbures aromatiques sur un catalyseur non acide afin de former de l'alkyl-cyclohexane ;
l'isomérisation de l'alkyl-cyclohexane afin de former des alkyl-cyclopentanes ; et
la déshydrogénation de l'alkyl-cyclohexane résiduel afin de former des hydrocarbures aromatiques dans un courant de produit (14), où le courant de produit comporte des hydrocarbures aromatiques et les alkyl-cyclopentanes ;
où le courant riche en hydrocarbures aromatiques possède une concentration initiale de 60% en poids d'hydrocarbures aromatiques, et où la déshydrogénation de l'alkyl-cyclohexane résiduel afin de former des hydrocarbures aromatiques dans un courant de produit forme le courant de produit ayant moins de 25% en poids d'hydrocarbures aromatiques.

2. Méthode selon la revendication 1, dans laquelle l'isomérisation de l'alkyl-cyclohexane afin de former les alkyl-cyclopentanes comprend l'isomérisation de l'alkyl-cyclohexane afin de former du diméthylcyclopentane.

3. Méthode selon la revendication 1, dans laquelle la saturation d'hydrocarbures aromatiques dans le courant riche en hydrocarbures aromatiques afin de former de l'alkyl-cyclohexane et l'isomérisation de l'alkyl-cyclohexane afin de former des alkyl-cyclopentanes comprend simultanément la saturation d'hydrocarbures aromatiques dans le courant riche en hydrocarbures aromatiques et l'isomérisation de l'alkyl-cyclohexane sur un catalyseur bifonctionnel afin de former de l'alkyl-cyclopentane.

4. Méthode selon la revendication 1, dans laquelle la déshydrogénation de l'alkyl-cyclohexane résiduel afin de former des hydrocarbures aromatiques dans un courant de produit comprend la déshydrogénation de l'alkyl-cyclohexane résiduel sur un catalyseur de déshydrogénation monofonctionnel.

5. Méthode selon la revendication 1, comprenant en outre le fractionnement du courant riche en hydrocarbures aromatiques à l'aide d'un reformat catalytique.

6. Méthode selon la revendication 1, comprenant en outre le fractionnement du courant riche en hydrocarbures aromatiques à l'aide d'un reformat catalytique, où le courant riche en hydrocarbures aromatiques comprend au moins 15% en poids de toluène, où le toluène est saturé dans le courant riche en hydrocarbures aromatiques afin de former du méthylcyclohexane, et où le méthylcyclohexane résiduel est déshydrogéné afin de former du toluène dans le courant de produit.

7. Méthode selon la revendication 6, dans laquelle la déshydrogénation du méthylcyclohexane résiduel afin de former du toluène dans un courant de produit forme le courant de produit avec un indice d'octane (RON) d'au moins 90.
